Europäisches Patentamt
European Patent Office
Office européen des brevets

(11) Publication number: **0 276 832 A2**

# EUROPEAN PATENT APPLICATION

(21) Application number: **88101178.7**

(22) Date of filing: **27.01.88**

(51) Int. Cl.$^4$: **C12P 7/60** , C12N 15/00 , C12N 1/20 , C12Q 1/68

(30) Priority: **30.01.87 US 8993**

(43) Date of publication of application: **03.08.88 Bulletin 88/31**

(84) Designated Contracting States: **AT BE CH DE FR GB IT LI NL**

(71) Applicant: **F. HOFFMANN-LA ROCHE & CO. Aktiengesellschaft**

**CH-4002 Basel(CH)**

(72) Inventor: **Kahn, Maria Sparacino**
**5025 Nicklas Place, N.E.**
**Seattle Washington 98105(US)**
Inventor: **Manning, Ronald Fred**
**3 Carlisle Drive**
**Livingston, N.J. 07039(US)**

(74) Representative: **Wuesthoff, Franz, Dr.-Ing. et al**
**Patentanwälte Wuesthoff -v. Pechmann-Behrens-Goetz Schweigerstrasse 2**
**D-8000 München 90(DE)**

(54) **Process for the preparation of cetogulonic acid.**

(57) Verfahren zur Produktion zur 2-Keto-L-gulonsäure aus Sorbose mittels rekombinanter Bakterien, einschliesslich der Expressionsvektoren, bei dieser Technik benützten Mutanten, rekombinanten Bakterien und Probenanordnungen.

Figur 2

Modell der Transposon-Mutagenese

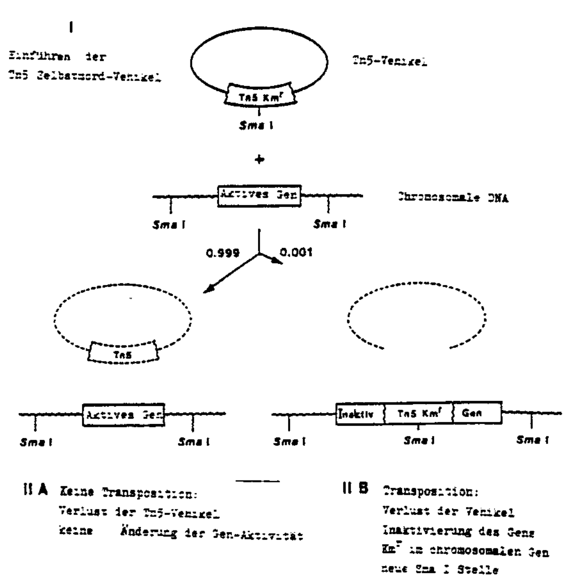


Xerox Copy Centre

EP 0 276 832 A2

# Verfahren zur Herstellung von Ketogulonsäure

Die Verbindung 2-Keto-L-gulonsäure (2-KGS) ist ein für die Synthese von Ascorbinsäure (Vitamin C) wichtiges Zwischenprodukt. Es ist bekannt, dass zahlreiche Mikroorganismen diese 2-Keto-L-gulonsäure, ausgehend von Sorbit oder L-Sorbose, produzieren, beispielsweise Mikroorganismen der Genera Acetobacter und Pseudomonas (siehe z.B. die Japanische Patentpublikation 40,154/1976). Zahlreiche Verbindungen sind als Zwischenprodukte dieser Synthese von 2-KGS, ausgehend aus L-Sorbose, bekannt geworden (Okazaki et al., Agr. Biol. Chem. 33, 207-211 [1969]). Die vorliegende Erfindung betrifft den Reaktionsweg über das Zwischenprodukt L-Sorbosone und die Nebenroute zu L-Idonsäure; Allerdings gelten die im vorliegenden entwickelten Prinzipien eigentlich für jeden Weg, der von L-Sorbose ausgeht und zu 2-KGA führt (Makeover et al., Biotechnol. und Bioeng. 17, 1485-1514 [1975]).

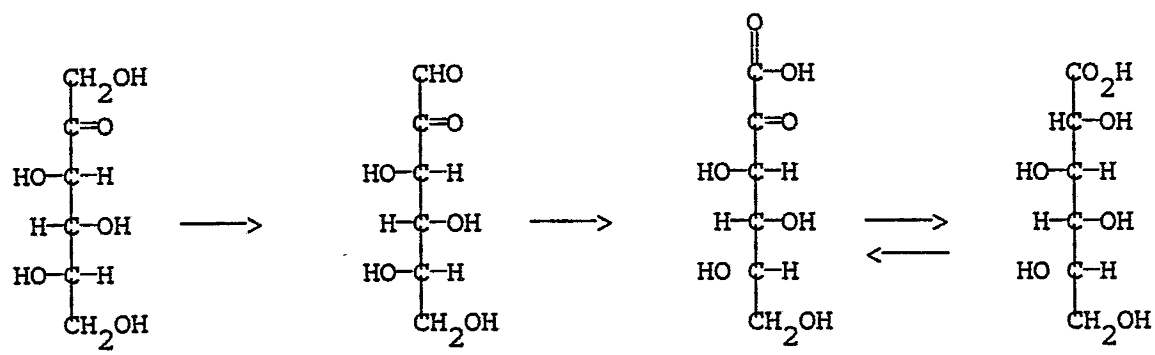

L-Sorbose    L-Sorbosone    2-Keto-L-gulon saeure    L-Idon saeure

Die mikrobiologische Konversion der L-Sorbose zu L-Sorbosone wird mittels des Enzyms L-Sorbosedehydrogenase bewerkstelligt, welches Enzym durch den eingesetzten Mikroorganismus produziert wird. Bei diesem mikrobiologischen Verfahren zur Herstellung der 2-KGS wird die Konversion der zunächst gebildeten L-Sorbosone zu 2-KGS durch das Enzym L-Sorbosonedehydrogenase bewerkstelligt, wiederum ein Enzym, das durch den Mikroorganismus produziert wird (U.S. Patent 3,907.639). Nun ist es aber so, dass viele Mikroorganismen, die die L-Sorbosonedehydrogenase produzieren, nicht auch das Enzym L-Sorbosedehydrogenase produzieren - wobei der umgekehrte Fall nicht eintritt.

Im Falle mehrerer Mikroorganismen wurde festgestellt, dass die Ausbeute an 2-KGS sinkt, dies ist zurückzuführen auf die Aktivität der 2-KGS-Reduktase, eines Enzyms, das 2-KGS zu L-Idonsäure reduziert.

Es existieren noch andere Reaktionswege für den Metabolismus von L-Sorbose durch die verschiedene Enzyme. Die meisten dieser Wege bedingen eine Erniedrigung der Ausbeute an 2-KGS. Es ist deshalb wünschenswert, diese Wege gänzlich zu eliminieren oder doch zu minimalisieren, was dadurch geschehen kann, dass man die Herstellung von kompetitiven Enzymen eliminiert oder minimalisiert, und so die Ausbeute an 2-KGS erhöht. Da 2-KGS selbst für das Bakterienwachstum nicht essentiell ist, ist es nicht möglich, auf diese Art direkt nach effizienten, mikrobiellen Produzenten zu suchen, zu "screenen". Bekannte mikrobielle Produzenten von 2-KGS konnten z.B. erhalten werden, indem man eine grosse Anzahl natürlicher Isolate untersuchte, siehe Guanglin et al., Acta Microbiologica Sinica 20, 246-251 (1980). Es ist deshalb wünschenswert, einfachere Methoden zu finden, um die Produktion von 2-KGS erhöhen zu können.

Im Rahmen der vorliegenden Erfindung wurde nun die Transposonmutagenese benützt, um zu einer Methode zu gelangen, 2-KGS mikrobiologisch herzustellen, und zudem die Produktion von Mikroorganismen, die fähig sind, die 2-Keto-L-gulonsäure aus L-Sorbose zu ermöglichen, zu erhöhen. Durch Einsatz der Transposonmutagenese im Rahmen der vorliegenden Erfindung war es möglich, das Screening von Tranposonmutanten stark zu vereinfachen, verglichen mit dem Screening konventioneller Mutanten. Zusätzlich hat sich die Transposoninsertion als Methode erwiesen, mit der ein interessantes Gen allein auf Basis seiner Funktion isoliert und geklont werden konnte. Ein solches Gen kann auch manipuliert werden, womit die Herstellung von 2-KGS mittels anderer Organismen beeinflusst werden kann. Es ist auf diese Weise möglich geworden, die Synthese von 2-KGS auf einfachere Weise als mittels konventioneller Methoden zu verbessern.

Im Rahmen der vorliegenden Erfindung werden verbesserte Methoden für die Mutagenese und das

Screenen von Organismen, die 2-KGS herstellen können, zur Verfügung gestellt. Auf diese Weise war es auch möglich, ein neues und verbessertes mikrobiologisches Verfahren zur Konversion von L-Sorbose zu 2-KGS zu entwickeln.

Im Rahmen der vorliegenden Erfindung wird auch eine Methode zur Verfügung gestellt, die es erlaubt, Gene, die für die Produktion von 2-KGS aus L-Sorbose wesentlich sind, zu identifizieren.

Im Rahmen der vorliegenden Erfindung wird auch eine Methode zur Verfügung gestellt, mit welcher rekombinante Plasmide mittels des Vektors RSF1010 mit breiter Wirtsspezifität konstruiert werden können. Schliesslich wird eine Methode zur Verfügung gestellt, um diese Plasmide in Gluconobacter oxydans oder andere Bakterien, beispielsweise in die in der Europäischen Patentpublikation Nr. 221 707 vom 13.5.1987 beschriebenen, einzuführen.

Diese Methoden erlauben, die DNS, die für die Herstellung von L-Sorbosedehydrogenase wichtige Produkte kodiert, zu identifizieren und zu klonen. Wenn rekombinante Plasmide mit einem Gehalt an der L-Sorbosedehydrogenase/DNS in einen Gluconobacter oxydans eingeführt werden, und zwar in einen, der keine 2-KGS produziert, erhalten diese Organismen die Fähigkeit, 2-KGS zu produzieren, und zwar mit der Ausbeute derjenigen Organismen, die die L-Sobosedehydrogenase-DNS lieferten. Demzufolge wird durch die vorliegende Erfindung eine verbesserte Methode zur Herstellung von 2-KGS zur Verfügung gestellt.

Die verschiedenen Aspekte der vorliegenden Erfindung können demgemäss wie folgt zusammengefasst werden:

Ein Verfahren zur Herstellung von 2-KGS aus Sorbose, das dadurch gekennzeichnet ist, dass man zur Konversion von Sorbose zu 2-KGS befähigte rekombinante Bakterienzellen in einem L-Sorbose enthaltenden Medium kultiviert.

Ein Verfahren zur Konversion von L-Sorbose zu 2-KGS, das dadurch gekennzeichnet ist, dass man in einem L-Sorbose enthaltenden Medium einen Mikroorganismus kultiviert, welcher Mikroorganismus eine Mutante eines natürlichen, zur Konversion von L-Sorbose zu 2-KGS befähigten Mikroorganismus ist, und, welcher Mutante eine Transposon in ihre zur Expression von 2-KGS-Reduktase befähigte DNS insertiert wurde, wodurch die Expression dieser Reduktase inaktiviert wird.

Eine Mutante eines zur Konversion von L-Sorbose zu 2-KGA befähigten Mikroorganismus, wobei der DNS, die verantwortlich ist für die Expression von Enzymen, die andere Zucker als 2-KGS produzieren, ein Transposon insertiert wurde, das die Expression dieser andern Enzyme inaktiviert.

Ein rekombinantes Bakterium, enthaltend Zellen mit rekombinantem Vektor mit der zur Expression der L-Sorbosedehydrogenase befähigten DNS-Sequenz.

Ein rekombinanter Vektor, der die DNS-Sequenz enthält, welche die Expression von L-Sorbosedehydrogenase ermöglicht.

Ein Verfahren zur Produktion von L-Sorbosehydrogenase, dadurch gekennzeichnet, dass man eine Wirtszelle mit einem Gehalt an rekombinantem Vektor zur Expression von L-Sorbosedehydrogenase kultiviert.

Eine Probenanordnung zur Bestimmung der Anwesenheit der DNS-Sequenz, die für L-Sorbosedehydrogenase kodiert, enthaltend einen Vektor mit radiomarkierter DNS-Sequenz, welche zur L-Sorbosedehydrogenaseexpression befähigt, wobei diese Sequenz durch Einfügen eines Transposons zur Expression der L-Sorbosedehydrogenase unfähig gemacht wurde.

## Legende zu den Zeichnungen

Aus der Figur 1 sind die Restriktionsendonukleaseschnittstellen von Tn5 ersichtlich; siehe in diesem Zusammenhang D. Berg und C. Berg, Biotechnologie 1, 417-435 (1983).

Die Figur 2 illustriert die Transposonmutagenese von Tn5.

Die Figur 3 illustriert die Konstruktion der Probenanordnung für das Gen, das die Expression von L-Sorbosedehydrogenase ermöglicht und die Konstruktion des Plasmids, das dieses Gen enthält.

Die Figur 4 ist eine Karte von RSF1010 mit den Restriktionsendonukleaseschnittstellen.

Die Figur 5 wiedergibt die Restriktionsendonukleaseschnittstellen der Gruppe 1-L-Sorbosedehydrogenase (Sorboseoxidase)-Genregion.

Die Figur 6 ist die Restriktionsendonukleaseregion der Gruppe 2-L-Sorbosedehydrogenase (Sorboseoxidase)-Genregion.

## Definitionen

Nukleotid.

Eine monomere Einheit der DNS, bestehend aus einer Zuckereinheit (einer Pentose), einem Phosphatrest und entweder einer Purin-oder Pyrimidinbase (also einem N-Heterocyclus). Die Base ist via das glycosidische Kohlenstoffatom (das 1′-Kohlenstoffatom der Pentose) mit dem Zuckerrest verbunden. Diese Art der Kombination einer Base und eines Zuckers nennt man, wie gesagt, ein Nukleotid. Jedes Nukleotid ist durch seine Base charakterisiert. Die vier in Frage kommenden Basen sind Adenin ("A"), Guanin ("G"), Cytosin ("C") und Thymin ("T").

DNS-Sequenz.

Eine lineare Anordnung von Nukleotiden, die miteinander über Phosphordiesterbindungen (zwischen dem 3′ und 5′Kohlenstoffatom benachbarter Pentosen) verbunden sind.

Genom.

Das gesamte genetische Material (DNS) einer Zelle oder eines Virus. Es enthält unter anderem die Strukturgene, die für die Polypeptide der Substanz kodieren, aber auch Operator, Promotor und ribosomale Bindungsstellen und Wechselwirkungs-Sequenzen, beispielsweise Sequenzen wie die Shine-Dalgarno-Sequenzen.

Struktur-Gen.

Eine DNS-Sequenz, welche über ihr Templat oder ihre Boten-RNS ("mRNS") eine Sequenz von Aminosäuren, die für ein bestimmtes Polypeptid charakteristisch ist, kodiert.

Transkription.

Verfahren, um aus einem Strukturgen die entsprechende mRNS zu produzieren.

Translation.

Verfahren, um das Polypeptid aus der mRNS zu produzieren.

Expression.

Verfahren, dem ein Strukturgen unterworfen wird, um das entsprechende Polypeptid zu erzeugen.

Plasmid.

Ein ringförmiges doppelsträngiges DNS-Molekül, nicht Teil des Hauptchromosoms eines Organismus, ein Molekül das Gene enthält, die Resistenz gegen spezielle Antibiotika bewirken. Wenn ein solches Plasmid in einen unizellulären Organismus plaziert wird, ändern sich die Charakteristika dieses Organismus, oder sie werden sogar transformiert, dies als Resultat der in dem Plasmid enthaltenen DNS. So transformiert beispielsweise ein Plasmid, das das Gen für die Tetracyclinresistenz ($Tet^R$) enthält, eine Zelle, die vorher empfindlich war auf Tetracyclin, in eine, die nun gegenüber diesem antibiotikum resistent wird. Eine Zelle, die durch ein solches Plasmid transformiert wurde, nennt sich Transformante, eine transformierte Zelle.

Klonierungsvehikel.

Ein Plasmid, Bakteriophagen-DNS oder andere DNS-Sequenzen, die fähig sind, in einer Wirtszelle zu replizieren. Sie sind gekennzeichnet durch eine oder eine kleine Anzahl von Endonukleasen-Erkennungssequenzen, bei denen diese DNS-Sequenzen ausgeschnitten werden können, und zwar auf eine ganz bestimmte, determinable Art, wobei dies ohne Verlust von biologischen Begleitfunktionen der DNS geschieht, z.B. Replikation, Produktion von viralen Hüllproteinen, oder Verlust von Promotor-oder Bindungsstellen. Die Vehikel enthalten einen Marker, der geeignet ist, transformierte Zellen zu identifizieren, Tetracyclinresistenz oder Ampicillinresistenz dienen beispielsweise diesem Zweck. Der Begriff Klonierungsvehikel wird oft mit dem Begriff Vektor synonym verwendet.

Klonieren.

Eine Methode zur Erzeugung einer Population von Organismen oder DNS-Sequenzen aus einem solchen Organismus mittels asexueller Reproduktion.

Rekombinantes DNS-Molekül, Hybrid-DNS.

Ein Molekül bestehend aus DNS-Segmenten verschiedener Genome, endständig und in vivo, also ausserhalb von lebenden Zellen, zusammengefügt. Das Molekül hat die Fähigkeit, eine Wirtszelle zu infizieren und sich dort zu erhalten.

Transposone sind eine Klasse von beweglichen genetischen Elementen, die nicht unabhängig replizieren können, die vielmehr in eine Wirtszelle insertiert werden müssen, wodurch die Propagation ermöglicht wird (N. Kleckner, Roth; und D. Botstein, J. Mol. Biol. 216, 125-159 [1977]. Diese Insertion in die Wirtszelle kann an einer Vielzahl von Stellen erfolgen (Shan, K. und Berg, C., Genetics 92, 741-747 [1979]. Es hat sich nun gezeigt, dass die Distribution der Insertionsstellen des Transposons Tn5 in vielen bakteriellen DNS viel zufälliger ist als dies im Falle anderer Transposone festgestellt wurde. Andererseits können aber auch für den vorliegenden Fall andere transposible DNS-Element, beispielsweise Tn10 oder der Bakteriophage Mu benützt werden. Allerdings ist Tn5 bevorzugt, siehe diesbezüglich Figure 1.

Wenn nun Tn5 in ein funktionsfähiges Gen insertiert wird, beispielsweise gemäss Figur 2, geht die Aktivität dieses Gens verloren, und es wird eine Transposonmutante kreiert.

Gemäss D Berg und C. Berg, Biotechnologie 1, 417-435 [1983] kodiert das Transposon Tn5 für diejenigen Aktivitä ten, die seine eigene Transposition regulieren. Zusätzlich enthält aber das Transposon Tn5 antibiotische Resistenzmarker für Kanamycin und Streptomycin. Aus diesem Grund können für die Transposonmanipulation selektive Verfahren benützt werden.

Die Transposonmutagenese ist sehr gut geeignet, um die Herstellung von "nicht-essentiellen" mikrologischen Produkten wie 2-KGS zu verbessern. Die Transposition eines Transposons in einer Zelle ist selbstlimitierend, und daraus folgt, dass eine Zelle selten mehr als eine Kopie eines bestimmten Transposons enthält. Auf diese Weise ist es auch möglich, transposonmutagenisierte Zellen auf einfache Weise zu screenen. Man benützt dazu die entsprechenden, dem Transposon eigenen Transposonantibiotikaresistenzmarker, um sicher zu stellen, dass nur mutagenisierte Zellen selektioniert werden. Zudem ermöglicht der selbstlimitierende Aspekt der Transposoninsertion, dass nur einzelne Mutanten gescreent werden. Mittels der Transposonmutation wird zudem eine sehr wertvolle Markierung an einem andererseits nicht selektierbaren Gen angebracht. Auf diese Weise markiert, kann das mutierte Gen isoliert werden, so dass sein Wildtyp geklont werden kann.

Im Rahmen der vorliegenden Erfindung kann deshalb das 5,7 kb lange Tn5 (Figur 1), das für Kanamycinresistenz ($Km^r$) in E.coli, aber auch für Streptomycinresistenz ($Sm^r$) in vielen anderen gramnegativen Erdbakterien kodiert, für die Konstruktion einer Probenanordnung für L-Sorbosedehydrogenase-DNS benützt werden.

Dieses Verfahren der Tn5-Mutagenese ist aus Figur 3 ersichtlich. Tn5 wird auf einem unstabilen (einem "Selbstmord"-Vehikel), das in der Zelle nicht aufrecht erhalten werden kann, eingefügt. Geeignete Vehikel sind R91-5 und pSup2021, allerdings ist das bevorzugte Vehikel der Bakteriophage P1 (Kuner und Kaiser, Proceedings of the National Academy of Sciences (USA), 78, 425-429 [1981]). In den meisten Zellen gehen das Vehikel und die Tn5 Partikel gleichzeitig verloren. Gelegentlich gelangt aber das Tn5 an eine Stelle in der Wirts-DNS, bevor das Vehikel verloren geht. Diese Stelle und damit die ganze Zelle besitzt nun den $Km^r$ Marker von Tn5. Wenn nun die Stelle innerhalb einer funktionalen DNS-Sequenz ist, geht die Aktivität

dieser Sequenz verloren. Gemäss einem bevorzugten Aspekt der vorliegenden Erfindung wird die Transposonmutagenese an einem G. Oxydans-Stamm, der ungefähr 20 g/l 2-KGS aus 70 g L-Sorbose erzeugen kann, durchgeführt. Die Identifikation des die 2-KGS produzierenden Organismen als Gluconobacter oxydans basiert auf der Beschreibung in "Bergey's Manual of Systematic Bacteriology", Vol. 1, 275-278 (1984). Ein geeigneter Wirt für den vorliegenden Zweck ist der Stamm UV10. Es kann aber im Prinzip jeder Stamm, der 2-KGS aus L-Sorbose produziert, für die vorliegenden Zwecke benützt werden.

Um die Zellen, die in diesem L-Sorbose → 2-KGA-Reaktionsweg mutagenisiert worden sind, zu identifizieren, werden Kanamycinresistente ($Km^r$)-Zellen bei 30°C in einem geeigneten Medium mit einem Gehalt an 7 Gewichtsprozent L-Sorbose und einer geeigneten Stickstoffquelle, kultiviert. Nach fünf Tagen werden die Fermentationsprodukte auf 2-KGS untersucht, aber auch auf andere Sorbosemetaboliten, unter Benützung von dem Fachmann bekannter Methoden.

Einzelne Gluconobacterreaktionswege, welche L-Sorbose oder ein Derivat davon benützen, treten in Wettbewerb mit der Produktion von 2-KGS. Einer dieser Wege ist die Reduk tion von 2-KGS zu L-Idonsäure (M. Ameyama und O. Adachi, Methods in Enzymology 89, 203-211 [1982]); Makover et al. 1975, supra). Wenn nun eine Tn5 Mutante vorliegt, die die Möglichkeit für diesen konkurrierenden Weg verloren hat, wird natürlich mehr 2-KGS produziert.

Aus diesem Grund erreicht man mittels Transposonmutagenese zwei Ziele, nämlich

a) Man verbessert die Fähigkeit eines Mikroorganismus, der befähigt ist, aus L-Sorbose 2-KGS zu produzieren, indem man eine Mutante dieses Mikroorganismus kreiert, eine Mutante, in der einer oder mehrere konkurrenzierende Reaktionswege blockiert sind. Dies wird bewerkstelligt, indem man die DNS, die ein Enzym exprimieren kann, das für die Nebenreaktionen verantwortlich ist, durch Insertion eines Transposons inaktiviert. Wie oben gesagt, bewirken diese Enzyme Nebenreaktionen, die zur Produktion von anderen Zuckern als 2-KGS führen, und die damit die Ausbeute an 2-KGS erniedrigen. Eines dieser Enzyme ist, wie oben gesagt, die 2-Keto-L-gulonsäurereduktase, welche 2-KGS zu L-Idonsäure reduziert. Durch konventionelles Screenen der Mutanten, die durch diese Transposonmutagenese entstanden sind, und die für niedrigere L-Idonsäureproduktion verantwortlich sind, kann man zu Mutanten des originären Sorbose-KGS-Stamms mit höherer Ausbeute an 2-KGS gelangen, indem nun das Transposon in die DNS, die für das Enzym 2-Ketogulonsäurereduktase kodiert, insertiert ist. Obschon auch konventionelle Mutagene fähig sind, das 2-KGS-Reduktasegen zu inaktivieren, ist das Screenen nach in solcher Weise blockierten Mutanten viel einfacher im Falle der Tn5-Mutanten, da ja im letzteren Fall nur solche Mutanten isoliert und gescreent werden, die die $Km^r$-Resistenz aufweisen. Diese Mutante sind im Prinzip immer Einzelmutanten.

b) Gewisse andere Tn5-Mutanten haben nun die Aktivität des Enzyms, das mit der Produktion von 2-KGS im Zusammenhang steht, verloren. Solche Mutanten können die Fähigkeit, L-Sorbosedehydrogenase zu produzieren, verlieren. Solche Mutanten sind eben solche mit Tn5-Insertion in die DNS, die benötigt wird zur Synthese von L-Sorbosedehydrogenase. Diese L-Sorbosedehydrogenase-DNS kann ein Strukturgen sein oder ein regulierendes Element, dessen Funktion für die Synthese von L-Sorbosedehydrogenase essentiell ist. Das Chromosom der Tn5 Mutante mit fehlender L-Sorbosehehydrogenaseaktivität oder irgend einer anderen Aktivität, die für die Produktion von 2-KGS essentiell ist, besteht aus der DNS eines Mikroorganismus, beispielsweise Gluconobacter, welche an einem Punkt der DNS durch ein Transposon, beispielsweise Tn5 unterbrochen wurde. Diese DNS kann aus der Mutantenzelle isoliert werden. Aus der isolierten DNS kann die Probenanordnung der vorliegenden Erfindung konstruiert werden. Die Probe wird konstruiert durch Kombination von DNS mit der durch Tn5 unterbrochenen L-Sorbosedehydrogenase-DNS mit einem geeigneten Vektor. Das rekombinante Plasmid kann durch Insertion in eine geeignete Wirtszelle, eine solche, die Reproduktion erlaubt, geklont werden.

Eine breite Palette von Wirt-Klonierungsvehikelkombinationen können für die Klonierung der doppelsträngigen DNS benützt werden. So sind beispielsweise geeignete Segmente von chromosalen, nicht chromosalen und synthetischen DNS-Sequenzen als Klonierungsvehikel geeignet, beispielsweise die bekannten bakteriellen Plasmide, wie die Plasmide ex E.coli, beispielsweise pBR322, Bakteriophagen-DNS und Vektoren, die aus einer Kombination von Plasmiden und Bakteriophagen-DNS abgeleitet sind, wie dies im Falle von modifizierten Plasmiden zur Aufnahme von Bakteriophagen-DNS zutrifft, oder andere Expressionskontrollsequenzen oder Hefeplasmide. Geeignete Wirte sind Mikroorganismen, Säugetierzellen, Pflanzenzellen, etc. Bevorzugt sind Mikroorganismen und Säugetierzellen. Bevorzugte Mikroorganismen sind Bakterien wie E.coli, Bacillus subtilis, Bacillus stearothermophilus und actinomyces. Natürlich sind nicht alle Wirt/Vektorkombinationen gleich effizient für den Klonierungsvorgang. Die spezielle Selektion einer Wirt/Klonierungsvehikelkombination ergibt sich eigentlich immer auf Grund des im Rahmen der vorliegenden Erfindung geoffenbarten Prinzips.

Weiterhin können im Falle jedes spezifischen Klonierungsvehikels verschiedene Stellen für die Insertion der doppelsträngigen DNS in Frage kommen. Diese Stellen ergeben sich im allgemeinen jeweils durch die

spezifische Restriktionsendonuklease, die verwendet wird. Im Fall von pBR322 wird zweckmässigerweise die Stelle ClaI zwecks Insertion der durch Tn5 unterbrochenen DNS benützt. Es sind schon viele solcher Stellen bei der Herstellung rekombinanter Plasmide benützt werden, und die jeweils geeigneten Stellen sind dem Fachman geläufig. Es ist klar, dass ein im Rahmen der vorliegenden Erfindung nützliches Klonierungsvehikel nicht unbedingt eine Restriktionsendonukleaseschnittstelle haben muss, um das gewählte DNA-Fragment einzubauen. Das Vehikel kann auch auf andere Weise mit dem fraglichen Fragment verknüpft werden:

Es sind diesbezüglich mehrere Methoden, um DNS-Sequenzen in ein Klonierungsvehikel zu insertieren, mit dem Ziel, rekombinante DNS zu gewinnen, bekannt. Solche Methoden beinhalten: direkte Verknüpfung, synthetische Linkermoleküle oder die Bildung von komplementären überlappenden Fragmenten durch Verwendung von terminaler Transferase, womit die 3'OH Termini verlängert werden, gefolgt durch Annellieren (Zusammenfügen) der überstehenden Enden mit dem Ziel, rekombinante Plasmide zu erzeugen.

Aus der Figur 3 ist ersichtlich, dass die Insertion der für die L-Sorbosedehydrogenase relevanten vektorgebundenen und durch Tn5 unterbrochenen DNS in eine Wirtszelle auf an sich bekannte Weise erfolgen kann. Eine bevorzugte Wirtszelle, insbesondere im Falle des Vektors pBR322, ist E.coli. Die transformierte Wirtszelle klont auf diese Weise das rekombinante Plasmid. Zur Herstellung der Probe, wie dies in Beispiel 3 illustriert ist, extrahiert man das Plasmid und radiomarkiert die DNS mittels konventioneller Methoden, beispielsweise mittels Nick-Translation oder Polynukleotid-Kinase-Markierung.

Die Probenanordnung gemäss der vorliegenden Erfindung kann in der Folge benützt werden, um verschiedene DNS-Fragmente zu screenen und dadurch festzustellen, ob sie unmutierte DNS-Sequenzen, die für die Expression der L-Sorbosedehydrogenase benötigt werden, aufweisen.

Zwecks Erstellung der Genbank der Figur 3, die benötigt wird zur Bestimmung der Anwesenheit von Genen, Gene die die Expression von bei der Herstellung von L-Sorbose zu 2-KGS relevanten Enzymen induzieren, wird die DNS einem Organismus, der zur Konversion von Sorbose zu 2-KGS befähigt ist, entnommen. Ein bevorzugter Organismus ist Gluconobacter oxydans UV10. Die betreffende DNS wird der Behandlung mit einer Endonuklease, die eine Vierbasenfrequenz, die für Gluconobacter oxydans charakteristisch ist, erkennt, geschnitten; ein bevorzugtes Enzym ist HhaI, welches die Sequenz GCGC erkennt. Das Ausmass der Restriktion wird durch an sich bekannte Methoden, beispielsweise Kontrolle der Zeit oder Menge der anwesenden Enzymaktivität in einer Restriktionsreaktion bewerkstelligt, und zwar so, dass die meisten der erzeugten DNS-Fragmente zwischen 5 und 10 kb lang sind, eine bevorzugte Länge für die Erstellung der Genbank; selbstverständlich können aber auch andere Längen zum Einsatz gelangen. Die DNS der bevorzugten Länge wird mittels an sich bekannter Methoden isoliert, beispielsweise durch Gelelektrophorese oder Auftrennung (Sedimentation) in einem Dichtegradienten. Das Enzym terminale Transferase (TdT) wird benützt, um eine Oligonukleotidextension der dC (Deoxycytidin)-Schwänze an die 3' Hydroxyenden der isolierten Gluconobacter oxydans/DNS zu bewerkstelligen. Eine bevorzugte Verlängerung beinhaltet 15-20 Basen.

Der für die Genbank der Figur 3 benützte Vektor ist einer, der einerseits in Gluconobacter, andererseits aber auch im Klonierungsorganismus, z.B. E.coli, replizieren kann. Der bevorzugte Vektor ist RSF1010 (K. Nagahari und K. Sakaguchi, 1978, J. Bact. 133, 1527-1529) (Figur 4), es kann aber im Prinzip jeder Vektor, der in Gluconobacter als auch in E.coli repliziert, zum Einsatz gelangen. Der Vektor RSF1010 wird mit einer Endonuklease, beispielsweise PvuII geschnitten, also einer Endonuklease, die eine Stelle, wie CAGCTG erkennt, eine Stelle, die nicht in einer Region des Plasmids, die für die Replikation oder die antibiotische Resistenz benötigt wird, liegt. Es wird also Terminale Transferase eingesetzt, um die 3'OH-Enden des Vektors zu verlängern, und zwar, mit Deoxyguanidinresten und auf eine Länge von 15-20 Basen.

Der 3'OH verlängerte Vektor und die Gluconobacter-DNS werden zwecks Erstellen rekombinanter Moleküle verknüpft, und zwar nach an sich bekannten Methoden, beispielsweise durch Erhitzen und hierauf langsames Kühlen des Gemisches in einer 100 mM Natriumchloridlösung. Die rekombinanten Moleküle werden nun für die Transfektion eines bevorzugten Wirtes, beispielsweise E.coli, benützt. Es sollte eine bestimmte Wildtypgluconobactersequenz auf 3000 solcher unabhängiger Rekombinanten mindestens einmal in der Bank vorhanden sein.

Die Figur 3 illustriert die Identifikation der Klone mittels der Probenanordnung in der Wildtypbank, welche dieses L-Sorbosedehydrogenasegen enthält. Um zu bestimmen, welche rekombinanten Klone in der Bibliothek die Wildtyp L-Sorbosedehydrogenase-DNS enthalten, werden die Klone mit der Probe aus Tn5 mutierter DNS hybridisiert und zwar nach an sich bekannten Methoden, beispielsweise Anellierung (Hybrisierung) von DNS-Filterreplika der Bank mit der Probe.

Die rekombinante Plasmide, enthaltend den Wildtyp der L-Sorbosedehydrogenase-DNS können nun in den Recipienten, also den Mikroorganismus, durch Transfektion oder auch Mobilisierung übertragen werden. Die in jedem einzelnen Fall bevorzugte Methode ist aus Okamura et al. 1985, Agric. Bio. Chem.

49:1011-1017 ersichtlich. Die Wildtyp L-Sorbosedehydrogenase-DNS, insertiert in einen geeigneten Vektor, beispielsweise RSF1010, kann nun in einen geeigneten Rezipienten durch konjugativen Transfer des Plasmides aus dem E.coli Wirt in den Rezipienten übertragen werden, beispielsweise wenn ein geeignetes Helferplasmid in dieser E.coli Wirtszelle vorhanden ist; es kann aber auch eine Drittzelle, die Plasmidtransferfunktionen aufweist, benützt werden. Die Konjugation kann in flüssigem Medium oder auf einem Festkörper durchgeführt werden, was auch immer im einzelnen Fall bevorzugt ist. Der bevorzugte Rezipient ist ein Organismus, beispielsweise IFO3293, welcher Rezipient L-Sorbosonedehydrogenaseaktivität aufweist. Das Helferplasmid kann ein unabhängiges Replikon, d.h. ein replizierender Vektor, sein oder in das Zellchromosom integriert sein. Ein bevorzugtes Helferplasmid ist RP4, aber auch andere, dem Fachmann bekannte Plasmide können hier Verwendung finden.

Nach dem Transfer in den Rezipienten kann das rekombinante Plasmid replizieren und zwar als freies Plasmid, oder Portionen des Plasmids können in die chromosomale oder Plasmid-DNS der Zelle übergehen.

Die klonierte L-Sorbosedehydrogenase-DNS kann nun im neuen Wirt funktionieren, dergestalt, dass sie ein Genprodukt, das der wirt für L-Sorbosedehydrogenaseaktivität benötigt, komplementiert. Dies kann ein Strukturprotein sein, welches L-Sorbosedehydrogenaseaktivität aufweist, ein Hilfsprotein oder ein regulierendes Element. Eine Wirtszelle mit L-Sorbosonedehydrogenaseaktivität, welche die zur Synthese der L-Sorbosedehydrogenase benötigte klonierte DNS, auf diese Weise erhalten hat, ist nun auch in der Lage, 2-KGS aus L-Sorbose zu produzieren.

Die Produktion der 2-KGS durch den Rezipienten kann schliesslich mittels Wachstum in einem Medium, das L-Sorbose enthält, bestimmt werden, wobei ein Test auf 2-KGS Verwendung findet.

In den untenstehenden Beispielen werden die folgenden Mikroorganismen eingesetzt:

| Stamm | Hinterlegung |
|---|---|
| Gluconobacter oxydans melanogenus UV10 | FERM-p No. 8422 |
| G.O. Suboxydans IF03293 | FERM-p No. 8356 |
| E. coli W3110 (P1::Tn5) | NRRL B-18148 (x) |
| E. coli 600 | ATCC 33525 |
| E. coli C600 (RSF1010) | NRRL B-18146 (x) |
| E. coli J53 (RP4) | NRRL B-18147 (x) |
| G. Oxidans M25-18 | NRRL B-18143 (x) |
| G. Oxidans M43-53 | NRRL B-18144 (x) |
| G. Oxydans M23-15 | NRRL B-18145 (x) |

(x) Neue Stämme, die am 2. Dezember 1986 unter den Stipulationen des Budapestervertrags hinterlegt worden sind. In der obigen Tabelle bedeutet das Wort FERM, dass der Stamm in der Agency of Industrial Science and Technology, Fermentation Research Institute, Japan hinterlegt wurde. Der Begriff NRRL bezeichnet einen Stamm, der im Northern Regional Research Center des United States Department of Agriculture Peoria, Illinois, hinterlegt wurde. ATCC steht für American Type Culture Collection, Rockeville, Maryland, USA.

## Beispiel 1

Der Coliphage P1::Tn5 und das Protokoll für dessen Benützung stammen von Dr. Claire Berg, University of Connecticut.

P1::Tn5 Transposonmutagenese in Gluconobacter

Eine Starterkultur von E.coli W3110 (P1::Tn5) wird 18 bis 20 Stunden bei 30°C in LB (Luria Broth)-Medium (10 g/l NaCl, 10 g/l Bacto Tryptone (Difco Bacto Peptone), 5 g/l Hefeextrakt, wachsen gelassen, wobei auf kontinuierliches Rühren geachtet wird. 1 ml der Kultur wird in 100 ml P1-Medium (LB-Medium) (10mM $MgCl_2$, 10 mg/ml Thymidin) hinein verdünnt und bei 30°C wachsen gelassen und zwar bis zu einem $A_{550}$-Wert von 0,092 (Absorption bei 550 nm; Schichtdicke 1 cm). Die Zellen werden 10 Minuten auf Eis gekühlt, 10 Minuten bei 6°C und 2500 Umdrehungen pelletiert (sedimentiert) und in 20 ml P1-Medium resuspendiert. Die Phagenproduktion wird zunächst 20 Minuten bei 42°C induziert, hierauf noch 90 Minuten bei 37°C. Zellysis wird durch die Zugabe von 0,5 ml Chloroform komplettiert, es wird vortexiert, d.h. heftig durchmischt und 5 Minuten bei Zimmertemperatur inkubiert. Die Zelltrümmer werden 15 Minuten bei 1000 Umdrehungen pelletiert. Die überstehende Flüssigkeit wird in einen zehnfachen Ueberschuss an Puffer MCB (10mM $MgCl_2$, 10mM $CaCl_2$) hinein verdünnt und als Phagensuspension benützt.

Die bevorzugte Empfängerzelle für P1::Tn5 ist Gluconobacter oxydans UV10, welcher 20-25 g/l 2-KGS aus 70 g/l Sorbose erzeugt. Eine Kultur dieses Rezipienten wird in MB-Medium ("Mannitol broth") [25 g/l Mannit, 5 g Hefeextrakt und 3 g/l Bactopepton] wachsen gelassen, und zwar 18-20 Stunden bei 28°C; hierauf wird 1 ml in 29 ml MB-Medium verdünnt und wachsen gelassen bis zu einer Absorption $A_{550}$ von 0,4. Die Zellen werden bei 2500 Umdrehungen und bei 6°C pelletiert und hierauf in 1,8 ml MCB suspendiert. Zwecks Infektion werden 0,1 ml Zellen mit 0,1 ml Phagensuspension gemischt und 60 Minuten bei 30°C inkubiert.

Die infizierten Zellen werden hierauf mit 0,8 ml MB-Medium verdünnt und 2 Stunden bei Zimmertemperatur stehen gelassen. Plattiert wird auf MBA [MB, 15 g/l Agar und enthaltend 50 µg/ml Kanamycin] und inkubiert wird 3-4 Tage bei 30°C. Die Zellen, die Kolonien bilden, enthalten Tn5 insertiert in das Chromosom.

Mutantenanalyse

Kanamycinresistente Mutantenkolonien werden in 5 ml Wachstumsmedium (10 g/l $CaCO_3$, 7,5 g/l Hefeextrakt, 70 g/l Sorbit als Kohlenstoffquelle, 0,5 g/l Glyzerin, 2,5 g/l $MgSO_4{\bullet}7H_2O$, 50 µg/l Km) 48 Stunden bei 27°C wachsen gelassen; 0,5 ml werden hierauf in 5 ml Medium M3B transferiert (70 g/l Sorbose, 0,05 g/l Glyzerin, 12 g/l $CaCO_3$, 2,5 g/l $MgSO_4$, 5 g/l Hefeextrakt), 15 g/l Maisquellwasser, 50 µg/l Km; man lässt 5 Tage bei 27°C wachsen.

Die Fermentationsprodukte werden analysiert:

1) Mittels high performance liquid chromatography (HPLC). 100 µl Aliquots der Kulturbrühe werden mit 2 ml 0,5% o-Phenylendiamindihydrochlorid als fluoreszierendem Marker gemischt und zwar 30 Minuten bei 90°C in einer verschlossenen Phiole. Die Chromatographie wird auf einem C 18 u bond pak (einer handelsüblichen HPLC-Kolonne) durchgeführt, die Kolonne hat die Dimensionen 30 cm Länge × 3,9 mm innerer Durchmesser; die mobile Phase ist Methanol/Wasser (75:25), die Geschwindigkeit ist 2 ml/Minute, der Druck ist 2000 psi (1 psi = 0,068 Atmosphären).

·2) Zusätzlich wird Dünnschichtchromatographie dergestalt durchgeführt, dass 1 µl der Kulturbrühe auf eine Silica gelplatte aufgebracht wird, es wird entwickelt mit n-Propylalkohol, Wasser, 1%-iger Phosphorsäure und Ameisensäure (100:25:2,5:0,25). Nach der Entwicklung werden die Platten mit Tetrazolblau oder Tetrabase nach an sich bekannten Methoden besprüht.

Nach Dünnschichtanalyse von 2000 UV10:Tn5-Mutanten stellte sich heraus, dass eine Mutante, nämlich M23-15, mehr 2-KGS als die Stammkultur (Elternkultur) erzeugte. Eine dünnschichtchromatographische Analyse der Fermentationsprodukte zeigte ferner, dass das Nebenprodukt Idonsäure in nur sehr geringen Mengen anwesend war.

Um diese Befunde zu bestätigen, wurden eine Oese der Stammkultur UV10 und die Mutante M23-15 über Nacht bei 30°C in 2 ml MB-Medium wachsen gelassen, ein Medium das zusätzlich 50 µl/ml Kanamycin enthielt; 100 µl der Kultur wurden hierauf in 10 ml M3B-Medium transferiert (70 g/l L-Sorbose, 10 g/l $CaCO_3$, 2,5 g/l $MgSO_4{\bullet}7H_2O$, 5 g/l Hefeextrakt, 0,5 g/l Glyzerin) es wurden 50 µg/ml Km zugegeben und 5 Tage in einer 50 ml-Flasche bei 30°C unter heftigem Rühren wachsen gelassen. Die Mengen an 2-KGS wurden quantitativ mittels HPLC gemessen. Um die L-Idonsäure zu analysieren, wurde der pH-Wert der Probe auf einen Wert zwischen 1,5 und 2,0 eingestellt und es wurde eine Stunde auf 80°C erhitzt, um so zum Produkt Iodonolacton zu gelangen. 1 ml der Lactonlösung wurde zu 2 ml einer gleichvolumigen Lösung von 4M Hydroxyaminhydrochlorid und 4M Natriumhydroxid gegeben. Die Absorption wurde bei

540nm gemessen und verglichen mit einem gleichermassen behandelten Wasserleerwert. Die Konzentration der L-Idonsäure wurde durch Vergleich mit einer Standardkurve ermittelt; die Resultate ergeben sich aus der Tabelle I.

Tabelle I

Produktion von 2-KGS und Idonsäure durch die Tn5-Mutante 23-15

| | 2-KGS | Idonsäure |
|---|---|---|
| UV10 | 19.6 g/L | 10.9 g/L |
| M23-15 | 33.3 g/L | 2.8 g/L |

Aus der Tabelle I ist ersichtlich, dass die Mutante M23-15 wesentlich mehr 2-KGS produziert als der Stamm UV10, gleichzeitig ist die Menge an Nebenprodukt L-Idonsäure wesentlich geringer.

Die L-Idonsäure wird durch die 2-KGS-Reduktase erzeugt, ein Ferment, welches 2-KGS reduziert, wobei NADPH (Nikotinsäureamidadenindinukleotidphosphat) als Cofaktor fungiert. Entsprechend wurde in der Folge die 2-KGS-Reduktase in M23-15 und im Stamm UV10 bestimmt.

Eine Oese der Kultur wurde bis zur Sättigung in 5 ml MB-Medium wachsen gelassen, und zwar ein Tag bei 30°C und 300 Umdrehungen. 2 ml dieser Kultur wurde in 100 ml MB-Medium in einen 500 ml Erlenmeyerkolben transferiert; es wurde ein Tag bei 30°C und 300 Umdrehungen wachsen gelassen. 20 ml dieser Brühe wurden für die Inokulation von je 2 Flaschen mit M3B-Medium (500 ml pro 2000 ml Behälter) benützt und man liess 3 Tage bei 30°C und 300 Umdrehungen wachsen. Die Brühe wurde hierauf 10 Minuten bei 4°C und 400 Umdrehungen zentrifugiert, um $CaCO_3$ zu entfernen, hierauf wurde innert 20 Minuten bei 4000 Umdrehungen die Zellen bei 4°C pelletiert (zentrifugiert). Die Zellen, also das Sediment wurden zweimal mit eiskalter steriler 0,85 %iger Natriumchloridlösung gewaschen.

3 g der Zellen wurden in 10 ml 10mM Kaliumphosphatpuffer (pH 7) resuspendiert und 2 Minuten in einem Biospec Products Bead Beater (einem Mixer) homogenisiert. Das Homogenisat wurde bei 8,800 $^{\times}$ g 10 Minuten bei 4°C zentrifugiert, um Zelltrümmer zu entfernen, schliesslich noch 60 Minuten bei 100'000 $^{\times}$ g und 4°C. Die überstehende Lösung wurde als die Cytosolenzymfraktion benützt. Es wurden nun die Einheiten (E) Enzymaktivität in Mikromol Produkt, die in einer Minute bei 25°C gebildet wurden, bestimmt. Dies geschah durch Messen der Aenderung der Absorption des reduzierten Pyridincofaktors bei 340mm in einem Reaktionsgemisch folgender Zusammensetzung: 1 ml 2-KGS (100 µl einer 10 mg/ml-Lösung in 50mm Kaliumphosphatpuffer pH 7), 50 µl 2 mg/ml NADPH in 50 mm Kaliumphosphatpuffer pH 7), 25 µl Cytosolenzymfraktion (das "Lösliche") und 625 µl 50M Kaliumphosphatpuffer pH 7. Die Konzentration an Protein wurde unter Verwendung des Bio Rad Protein Assaygeräts gemessen.

Tabelle II

| | | |
|---|---|---|
| Spezifische Aktivität der 2-KGA-Reduktase E/mg Protein | UV 10 | 0,0342 |
| | M23-15 | 0,000067 |

Aus der Tabelle II ist klar ersichtlich, dass die 2-KGS-Reduktaseaktivität von M23-15 viel kleiner ist als diejenige von UV10. Dadurch wird bestätigt, dass die vorliegende Methode erlaubt, einen Mikroorganismus zu erhalten, der 2-KGS viel effizienter produziert als der ursprüngliche Stamm.

Beispiel 2

Klonen der in der L-Sorbosedehydrogenasesynthese involvierten Gene

Die Tn5-Mutagenese des 2-KGS produzierenden Gluconobacter oxydans UV10 und eine darauffolgende Analyse von 5000 Mutanten gestatteten die Isolierung zahlreicher Stämme, die weniger 2-KGS als der ursprüngliche Stamm produzierten. Vier dieser Stämme produzierten weniger als 1 g/l 2-KGS.

In diesen Stämmen hatte Tn5 eine für die 2-KGS-Produktion essentielle DNS-Sequenz inaktiviert. Zwei Enzymaktivitäten sind bei der Herstellung von 2-KGS durch den Stamm UV10 involviert: L-Sorbosedehydrogenase, die L-Sorbose zu L-Sorbosone oxidiert, und L-Sorbosonedehydrogenase, die L-Sorbosone weiter zu 2-KGS oxydiert. Um zu unterscheiden, welche dieser Aktivitäten nun fehlt, wurden wachstumskulturen der Mutante und Wildtypzellen in MB-Medium ein Tag bei 30°C wachsen gelassen. Je 2 ml dieser Wachstumskulturen wurden benützt, um 100 ml M3B Medium, 50 µg/l Km, in einem 500 ml Kolben zu inokulieren. Nach 2 Tagen bei 30°C wurden 25 ml der Zellkultur mittels Eis gekühlt, pelletiert, zweimal mit 40mM des Puffers ADA [N-(2-Actamido)-iminodiessigsäure] bei pH 6,8 und bei 4°C gewaschen, hierauf in 20mM ADA suspendiert (pH 6,8), welches ADA entweder 7% L-Sorbose oder 2% L-Sorbosone enthielt; inkubiert wurde bei 30°C und zwar 2 Tage und unter heftiger Belüftung. Die Produktion von 2-KGS wurde mittels HPLC bestimmt; die Resultate sind aus der untenstehenden Tabelle III ersichtlich.

Tabelle III

Produktion von 2-KGS ex L-Sorbose oder L-Sorbosone durch nichtwachsende Zellen (stationäre Kulturen)

| | 2-KGS (g/l) | |
|---|---|---|
| | aus L-Sorbose | aus L-Sorbosone |
| UV 10 | 8.3 | 6.1 |
| M2518 | 0 | 2.7 |
| M4353 | 0 | 3.8 |
| M4191 | 0 | 2.9 |

Es ist aus dieser Tabelle ersichtlich, dass die Kontrollzellen (Wildtyp) sowohl L-Sorbose als auch L-Sorbosone leicht zu 2-KGS konvertieren können, währenddem die Mutanten nur L-Sorbosone in 2-KGS überführen können. Die Mutantenzellen haben kein L-Sorbosonedehydrogenase-defizit, aber einen L-Sorbosedehydrogenasedefekt.

Um die mangelnde L-Sorbosedehydrogenaseaktivität zu bestätigen, wurden die Enzymspiegel bestimmt. Eine Oese von UV10 M43-53, M25-18 und M47-118 wurde jeweils benützt, um 15 ml MB-Medium zu inokulieren. Die stationären, 1 Tag-Kulturen wurden in 100 ml MB inokuliert, 1 Tag wachsen gelassen, und dann inokuliert und wachsen gelassen in M3B, wie in Beispiel 1 beschrieben. Die Zellen wurden geerntet und homogenisiert, wie im vorhergehenden Beispiel beschrieben und zwar nach Zentrifugation (100'000 g) des Homogenisats. Hierauf wurden die pelletierte Fraktion als Membranfraktion gesammelt. Die Membranfraktion wurde in 4 ml 10mM Kaliumphosphatpuffer pH 7 suspendiert. Zur Solubilisierung wurden 0,6 ml der Membransuspension mit 2,4 ml 10mM Kaliumphosphatpuffer pH 7 gemischt, einem Puffer, der 0,3% Triton X-100 enthielt, und es wurde 3 Stunden in 50 ml Puffer gerührt und dann eine Stunde zentrifugiert (100'000 g). Die überstehende Lösung wurde als solubilisierte Membranfraktion bezeichnet. Der Enzymtest wurde durch Messen der Abnahme der Absorption (bei 600nm) des Elektronenacceptors 2,6-Dichlorphenolindophenol (DCIP) durchgeführt. Eine Aktivitätseinheit wird dabei definiert als die Menge Enzym, die die Reduktion von 1 µMol von DCIP per Minute bei 25°C katalysiert. Die Testlösung enthielt 280 µl 0,1M Kaliumphosphatpuffer pH 7, 0,3% Triton X-100, 40 µl 2,5mm DCIP, 200 µl 1M L-Sorbose und 10 µl Enzymlösung und schliesslich 470 µl Wasser. Die Proteinkonzentration wurde gemäss Beispiel 1 bestimmt.

Tabelle IV

Spezifische Aktivität der Membrane-gebundenen L-Sorbose Dehydrogenase E mg Protein UV 10 0.194

| | |
|---|---|
| M25-18 | 0.023 |
| M43-53 | 0.013 |
| M47-118 | 0.007 |

Den Mutantenzellen fehlt klarerweise die L-sorbosedehydrogenaseaktivität, was so gewertet werden muss, dass eine Gluconobacter DNS-Sequenz, die für die Synthese von L-Sorbosedehydrogenase essentiell ist, nun inaktiviert worden ist.

Klonen der Tn5 Mutante

Die gesamte DNS wurde aus den Mutantenstämmen nach an sich bekannten Methoden isoliert. Die DNS wurde mit verschiedenen Restriktionsenzymen gemäss Tabelle V verdaut, durch Elektrophorese auf 1% Agarose fraktioniert und auf Nitrozellulosefilter übertragen (transferiert), wobei dies alles nach an sich bekannten Methoden geschah (Maniatis et al., Molecular Cloning, a Laboratory Manual, Cold Spring Harbor Laboratory Press, Cold Spring Harbor, 1982). Die übertragene DNS wurde an ColEI::Tn5 DNA anneliert, welch letztere mit 32P markiert worden war, und zwar durch Nick-Translation, und hierauf homologe Fragmente durch Autoradiographie nachgewiesen.

Die Längen der DNS-Fragmente, die an die Tn5-Probe annelierten, wurden berechnet, die entsprechenden Werte sind in der untenstehenden Tabelle V wiedergegeben.

Tabelle V

Restriktionsanalyse der DNS aus L-Sorbosedehydrogenase-defizienten Tn5 Mutanten

| | Fragment-Länge | | (Summe der Längen) |
|---|---|---|---|
| | Cla I | Eco RI | Bam HI |
| Mutante | | | |
| M25-18 | 16 Kb | 16.5 Kb | 5.0 |
| | | | 8.2 (13.2) |
| M38-97 | 16 | 16.5 | 5.2 |
| | | | 8.5 (13.7) |
| M41-91 | 9.2 | 27 | 7.6 |
| | | | 8.5 (16.1) |
| M43-53 | 9.2 | 27 | 7.0 |
| | | | 9.5 (16.5) |

Es resultierten klarerweise 2 verschiedene 2-KGS Mutanten; die Einteilung erfolgt gemäss Lokalisierung von Tn5: M25-18 und M38-97 sind in Gruppe 1, M43-53 und M41-91 sind in Gruppe 2.

Die Region der chromosomalen DNS aus M25-18 und M43-53, welche durch Tn5 inaktiviert wurde, wurde in E.coli kloniert, wobei der Vektor pRB322 benützt wurde und im übrigen wie folgt vorgegangen wurde: Die Mutanten-DNS wurde mit Cla 1 enzymatisch gespalten und hierauf an 0,7%-igem Agarosegel elektrophoresiert. Die DNS der richtigen Grösse wurde aus dem Gel ausgeschnitten und eluiert. In jedem Fall wurden ca. 200 ng Mutanten-DNS erhalten. Ungefähr 2 µg der pBR322-DNS wurde an ihrer einzigen Cla I-Stelle geschnitten und nach an sich bekannten Methoden mit alkalischer Phosphatase aus

Kälberserum behandelt. Mutante und Vektor-DNS wurden mittels T4-DNS Ligase verknüpft (verklebt) und die rekombinanten Plasmide wurden benützt, um den E.coli-Stamm C600 zu transformieren. Da Tn5 das Neomycinphosphotransferasegen enthält, wurden die rekombinanten Zellen auf Grund von $Km^r$ selektioniert.

Die Plasmide wurden aus den $Km^r$ Transfektanten durch eine Schnellmethode, basierend auf alkalischer Lysis erhalten (Birnboim and Doly, Nuc. A. Research 7, 1513-1523 [1979]). Die Plasmide wurden geschnitten und elektrophorisiert, um zu bestätigen, dass die korrekten Fragmente geklont wurden. Der Klon pM25-18 enthält. die mutierte Gruppe 1-L-Sorbosedehydrogenasesequenz: pM43-53 enthält diejenige der Gruppe 2.

Um nun den Klon der entprechenden Wildtyp-DNS zu erhalten, wurde eine Genbank der unmutierten DNS des Stammes UV10 hergestellt und zwar unter Verwendung eines Vektors mit breiter Wirtsspezifität, nämlich RSF1010. Ungefähr 200 µl der gesamten DNS ex UV10 wurden partiell mit ungefähr 10 Einheiten Hhal verdaut, und zwar eine Stunde; durch Gelelektrophorese und Eluieren wurden Fragmente von 5-10 kb Länge erhalten. Es wurde terminale Transferase (TdT) zur Verlängerung der 3'OH-Enden der Fragmente mit 15-20 Deoxycytidinresten benützt. Umgefähr 5 µg RSF1010 wurden mittels Restriktion mit Pvull linearisiert. Die 3'OH-Enden des Vektors wurden hierauf um 15-20 Deoxyguanidinreste ver längert. Je 100ng der nun komplementären Enden des Vektors und des Inserts wurden im Verhältnis 1:1 3 Stunden annelliert, dies bei 65°C in 100 µl 150mM NaCl, 10mM Tris vom pH 7,9 und 0,1mM AeDTA; hierauf wurde langsam auf Raumtemperatur abgekühlt und 20 µl des Produkts wurden benützt, um E.coli C600 zu transfizieren.

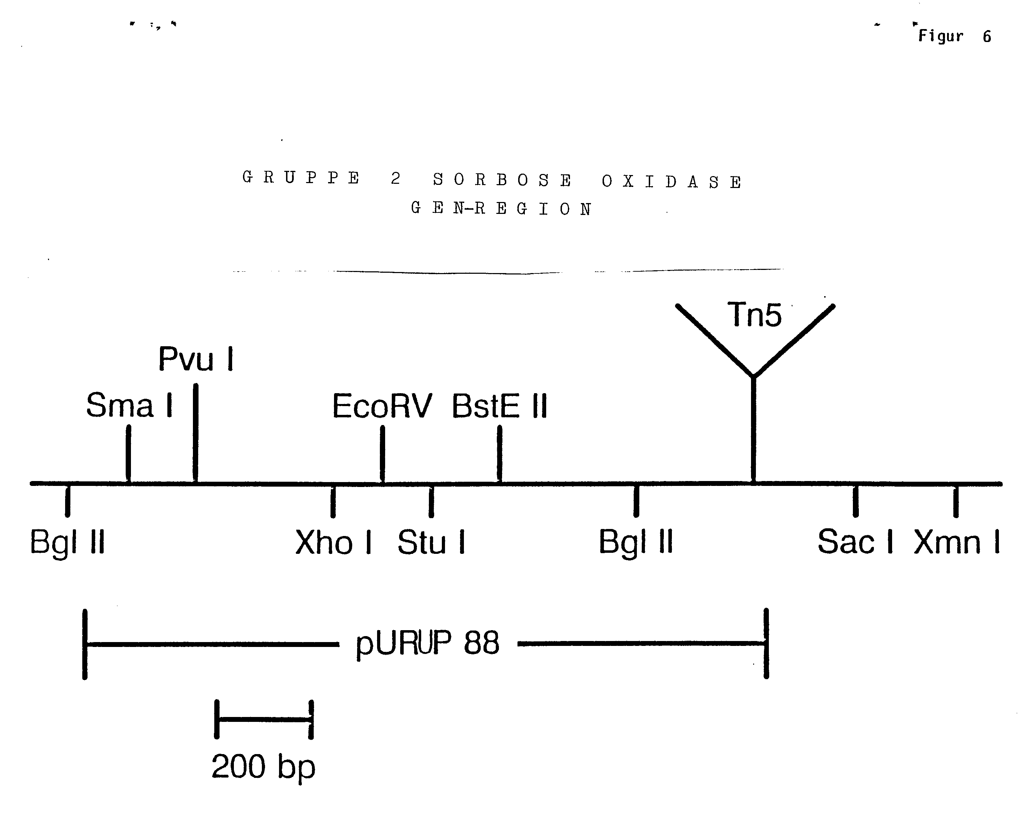

Nach Selektion auf Streptomycin enthaltendem LB-Agar wurden ungefähr 3000 rekombinante Kolonien erhalten. Die DNS einer Replika der Kolonien wurde in situ auf ein Set Nylonmembranfilter transferiert, und zunächst an 32p markiertem pM25-18 anneliert. Es wurden 2 verschiedene Gruppen 1-Rekombinanten erhalten, nämlich pURUH80 und pURU010, siehe diesbezüglich die Figur 5. Die radiomarkierte DNS der Filter wurde durch Erhitzen auf 90°C in einer Lösung von 10mM Tris (Tris(hydroxymethyl)-amino-methan) vom pH 7,5 und 1 ml AeDTA entfernt. Die filtergebundenen DNS-Kolonien wurden mit 32p pM43-53 rehybridisiert. Auf diese Weise wurde eine einzige Gruppe 2-Rekombinante, nämlich pURUP88 erhalten (Figur 6). Gemäss der vorliegenden Erfindung kann man demgemäss die Wildtypgluconobacter-DNS, die für die Herstellung der L-Sorbosedehydrogenase essentiell ist, klonieren.

## Beispiel 3

### Transfer der rekombinanten Plasmide, die L-Sorbosedehydrogenase DNS enthalten, auf 2KGS nicht-produzierende Gluconobacterstämme

Die rekombinanten Plasmide wurden auf den Stamm IFO 3293 übertragen, ein Stamm, der ca. 0-2 g/l 2-KGS aus 70 g/l L-Sorbose erzeugt, und von dem bekannt ist, dass er L-Sorbosonedehydrogenaseaktivität aufweist. Ein spontanes nalidixinsäureresistentes Derivat (Nar) von IFO3293 wurde isoliert durch Plattieren von 50 µl einer stationären MB-Medium-kultur auf eine Platte von MB-Ager, 100 µg/ml Na und Isolieren einer einzigen Kolonie. Der rekombinante Plasmidtransfer wurde durchgeführt durch Kreuzung mit 3 Elternstämmen (triparenterales Mating), wobei E.coli J53 (RP4) als Helferstamm fungierte. Ungefähr 200 µl Stationärphasenkulturen des Rezipienten IFO 3293, gewachsen in FB-Medium (25 g/l Fruktose, 5 g/l Hefeextrakt, 5 g/l Bactopepton) wurden mit ungefähr 100 ml das Donators E.coli C600 (pURUH80, pURUO10 oder pURUHP88) gemischt und die Helferstämme E.coli J53 (RP4) wurden in LB-Medium RP4 bis zu der sogenannten Midlogphase (in der Mitte der log-Phase, also in der Region des höchsten Wachstums) wachsen gelassen. Ungefähr 75 µl des Gemisches wurde auf einen Nitrocellulosefilter von 87 mm Durchmesser auf der Oberfläche einer Platte FB-Agar (FB, 15 g/l Agar) transferiert. Nach ungefähr 16 Stunden bei 30°C wurden die Kolonien auf FB-Agar aus gestrichen, Agar das 10 µl des Antibiotikum Polymixin B, 50 µg/l Na und 100 µ/l Streptomycin enthielt; es wurde 4-6 Tage bei 30°C weiterinkubiert. Auf diese Weise wurden nur diejenigen Gluconobacter IFO3293-Kolonien erhalten, welche die gewünschten rekombinanten Plasmide erhalten hatten. Diese Kulturen wurden in FB-Medien bei 30°C wachsen gelassen, und zwar 4 Tage. Hierauf wurden 2 ml dieser Keimkulturen in 100 ml M3B-Medium transferiert, ein Medium das 100 µg/ml Streptomycin enthielt, und es wurde unter dauerndem Rühren 5-7 Tage bei 30°C kultiviert. Die 2-KGS-Bestimmung wurde gemäss Tabelle VI durchgeführt.

Tabelle VI

Produktion von 2-KGS mittels rekombinanter Bakterien

| Wirt | Plasmid | 2KGS(g/l) |
|---|---|---|
| E. coli C600 | ---- | 0 |
| | RSF1010 | 0 |
| | PURUH80 | 0 |
| | PURUO10 | 0 |
| | PURUP88 | 0 |
| Gluconobacter IFO3293 | ---- | 1.8 |
| | RSF1010 | 0.5 |
| | PURUH80 | 25 |
| | PURUO10 | 28 |
| | PURUP88 | 11.8 |

Es ist ersichtlich, dass die L-Sorbosedehydrogenasegenbezogenen rekombinanten Plasmide den 2-KGS-Mangel von IFO3293 komplementiert haben. Um diese Behauptung zu bestätigen, wurden Enzymbestimmungen der rekombinanten Kulturen, wie oben angeführt, durchgeführt. Gemäss Tabelle VII weisen die rekombinanten Gluconobacterstämme eine L-Sorbosedehydrogenaseaktivität auf, die mindestens vergleichbar ist derjenigen des Stammes UV10 und wesentlich höher als diejenigen des zugrundeliegenden Stammes IFO3293 ist.

Tabelle VII

L-Sorbose-Dehydrogenaseaktivität von rekombinanten Gluconobacter-stämmen

| | |
|---|---|
| UV10 | 0.194 U/mg |
| IFO3293 | 0.019 |
| IFO3293 (pURO10) | 0.2 |
| IFO3293 (pURP88) | 0.13 |

**Claims**

1. Verfahren zur Herstellung von 2-Keto-L-gulonsäure (2-KGS) aus Sorbose, dadurch gekennzeichnet, dass man zur Konversion von Sorbose zu 2-KGS befähigte rekombinante Bakterienzellen in einem L-Sorbose enthaltenden Medium kultiviert.

2. Verfahren nach Anspruch 1, dadurch gekennzeichnet, dass das benützte Bakterium aus einem zur Konversion von Sorbose zu 2-KGS nicht befähigten Wirtsbakterium hergestellt wurde, wobei dem Wirt ein Vektor insertiert wurde, welcher den Wirt zur Konversion von Sorbose zu 2-KGS befähigt, und wobei dieser Vektor durch Transposonmutagenese erhalten wurde.

3. Verfahren nach Anspruch 1, dadurch gekennzeichnet, dass das rekombinante Bakterium durch ein wirtsbakterium gebildet wird, welch letzteres zwar L-Sorbosonedehydrogenase exprimieren kann, andererseits aber zur Expression von L-Sorbosedehydrogenase nicht befähigt ist, wobei der Wirtszelle ein Vektor insertiert wurde, der die DNS-Sequenz zur Expression von L-Sorbosedehydrogenase enthält.

4. Verfahren nach Anspruch 1, dadurch gekennzeichnet, dass die Bakterienzellen vom Genus Gluconobacter sind.

5. Verfahren nach Anspruch 2, dadurch gekennzeichnet, dass der Vektor, dem die DNS insertiert wurde, RSF1010 ist.

6. Verfahren zur Konversion von L-Sorbose zu 2-KGS gemäss Anspruch 1, dadurch gekennzeichnet, dass man in einem L-Sorbose enthaltenden Medium einen Mikroorganismus, und zwar eine Mutante eines natürlichen, zur Konversion von L-Sorbose zu 2-KGS befähigten Mikroorganismus, kultiviert, welcher Mutante ein Transposon in ihre zur Expression von 2-KGS-reduktase befähigten DNS insertiert wurde, wodurch die Expression dieser Reduktase inaktiviert wird.

7. Verfahren nach Anspruch 6, dadurch gekennzeichnet, dass das Transposon Tn5 ist.

8. Verfahren nach Anspruch 6, dadurch gekennzeichnet, dass der Mikroorganismus vom Genus Gluconobacter ist.

9. Mutante eines zur Konversion von L-Sorbose zu 2-KGS befähigten Mikroorganismus, wobei der zur Expression von anderen Zuckern als 2-KGS bildenden Enzymen befähigten DNS ein Transposon insertiert wurde, das die Expression dieser anderen Enzyme inaktiviert.

10. Mutante gemäss Anspruch 8, dadurch gekennzeichnet, dass der zur Expression von 2-KGS-reduktase verantwortlichen DNS ein Transposon insertiert wurde, das die Expression dieses Enzyms inaktiviert.

11. Mutante gemäss Anspruch 10, dadurch gekennzeichnet, dass das Transposon Tn5 ist.

12. Mutante gemäss Anspruch 10, dadurch gekennzeichnet, dass der Mikroorganismus vom Genus Gluconobacter ist.

13. Rekombinantes Bakterium, enthaltend Zellen mit rekombinantem Vektor mit der zur Expression der L-Sorbosedehydrogenase befähigten DNS-Sequenz.

14. Rekombinantes Bakterium gemäss Anspruch 13, worin die Zellen vom Genus Gluconobacter sind, welch letztere zur Produktion von L-Sorbosonedehydrogenase befähigt sind, nicht aber zur Produktion von L-Sorbosedehydrogenase.

15. Rekombinantes Bakterium gemäss Anspruch 13, worin der Vektor RSF1010 ist, welchem die zur Produktion von L-Sorbosone verantwortliche DNS insertiert wurde.

16. Das rekombinante Bakterium gemäss Anspruch 15, wobei dieses rekombinante Bakterium befähigt ist, L-Sorbose in 2-KGS zu konvertieren.

17. Rekombinanter Vektor, enthaltend die zur Expression von L-Sorbosedehydrogenase benötigte DNS-Sequenz.

18. Der rekombinante Vektor gemäss Anspruch 17, worin der rekombinante Vektor RSF1010 ist, welch letzterem die DNS-Sequenz zur Expression der L-Sorbosedehydrogenase insertiert wurde.

19. Verfahren zur Herstellung von L-Sorbosedehydrogenase, dadurch gekennzeichnet, dass man eine Wirtszelle mit rekombinantem Vektor, der zur Expression von L-Sorbosedehydrogenase befähigt ist, kultiviert.

20. Verfahren gemäss Anspruch 19, worin die Wirtszellen aus einem Mikroorganismus des Genus Gluconobacter sind, welcher ursprünglich die Fähigkeit hatte, L-Sorbosonedehydrogenase, nicht aber L-Sorbosedehydrogenase, zu exprimieren.

21. Verfahren gemäss Anspruch 19, worin der rekombinante Vektor RSF1010 ist, welch letzterem die DNS zur Expression von L-Sorbosedehydrogenase insertiert wurde.

22. Eine Probenanordnung zur Bestimmung der Anwesenheit der DNS-Sequenz, die für L-Sorbosedehydrogenase kodiert, enthaltend einen Vektor mit radiomarkierter DNS-Sequenz, welche zur L-Sorbosedehydrogenase-Expression befähigt ist, wobei diese Sequenz durch Einfügen eines Transposons zur Expression der L-Sorbosedehydrogenase unfähig gemacht wurde.

EP-62 288
RAN 4226/78

Figur 1

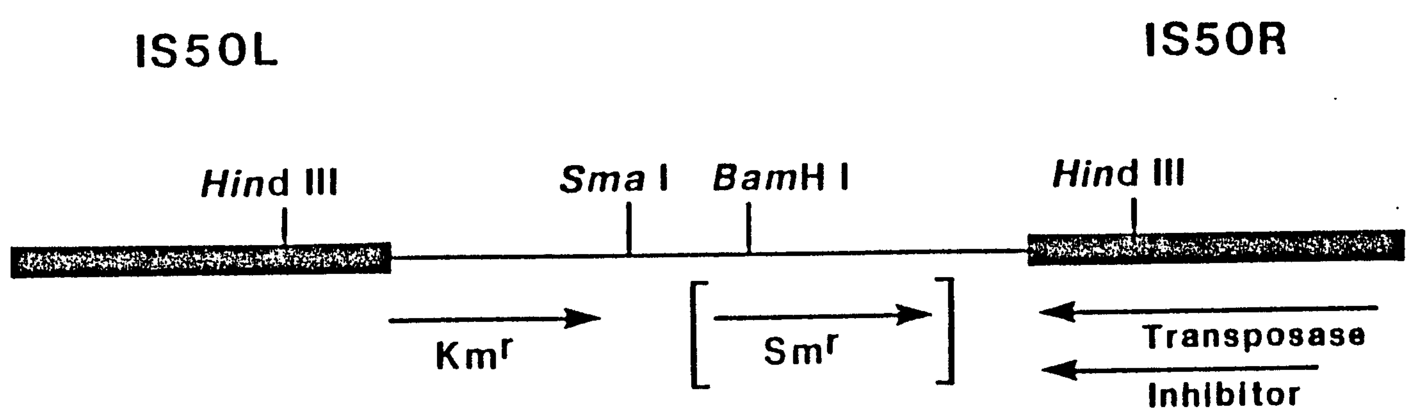


**Tn5 (5.7kb)**; Erkennungsstellen der Endonukleasen

D. Berg und C. Berg, Biotechnology 1, 417-435 /1983)

Figur 2

Modell der Transposon-Mutagenese

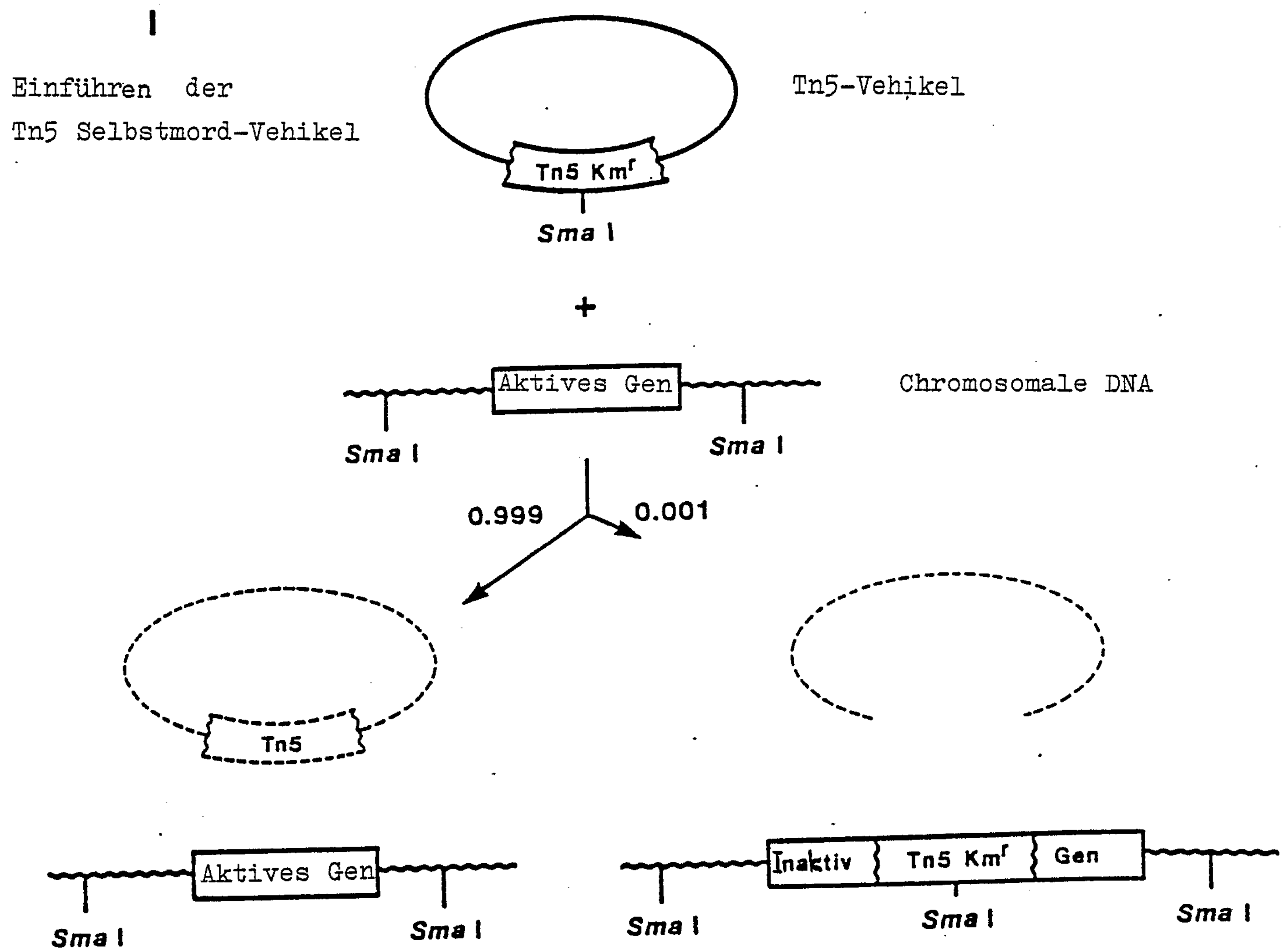


**II A** Keine Transposition:
Verlust der Tn5-Vehikel
keine Änderung der Gen-Aktivität

**II B** Transposition:
Verlust der Vehikel
Inaktivierung des Gens
$Km^r$ im chromosomalen Gen
neue Sma I Stelle

Figur 3

Verwendung der Tn5-Mutante zum Klonieren funktionsfähiger Gene aus Gluconobacter

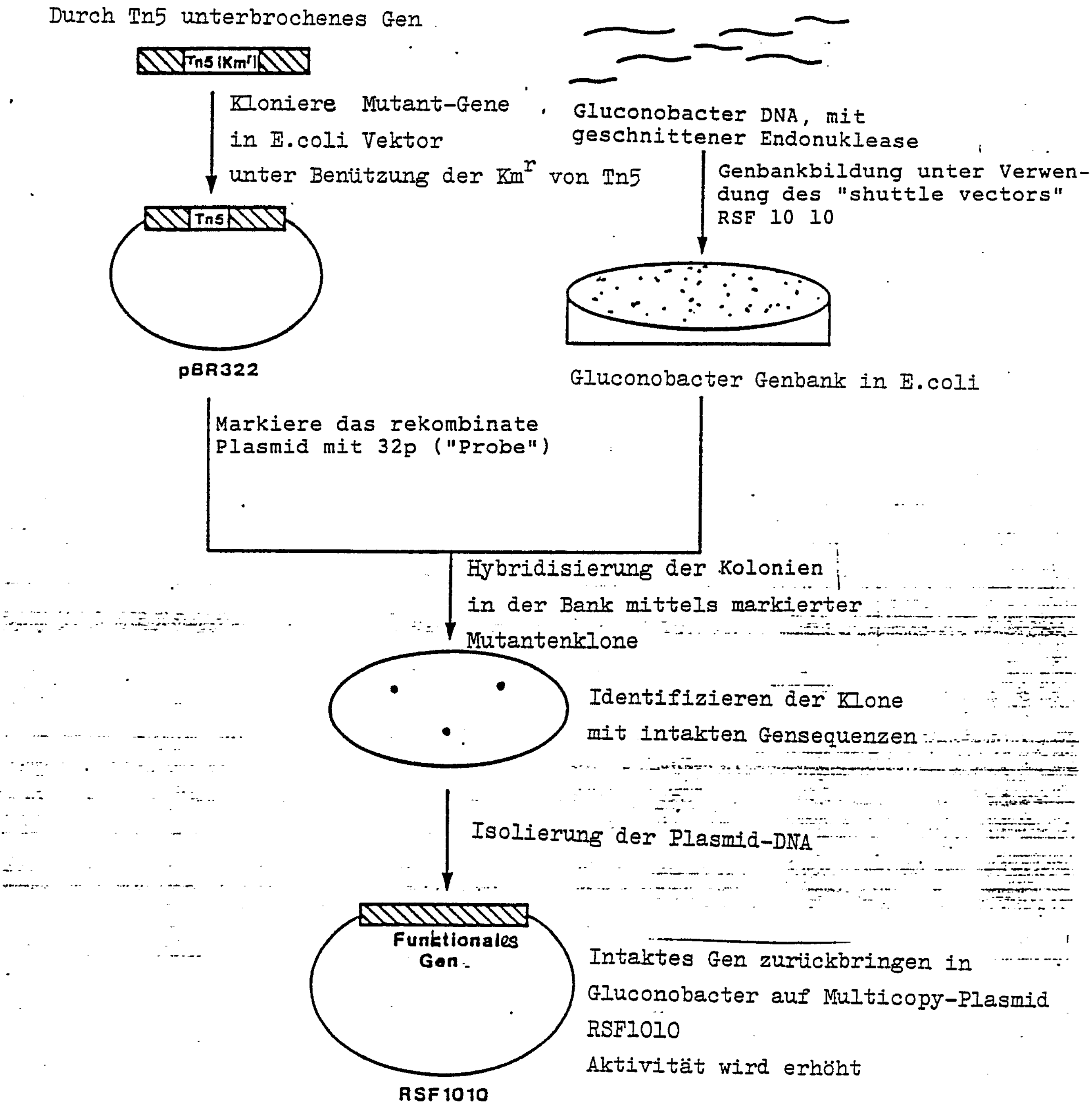

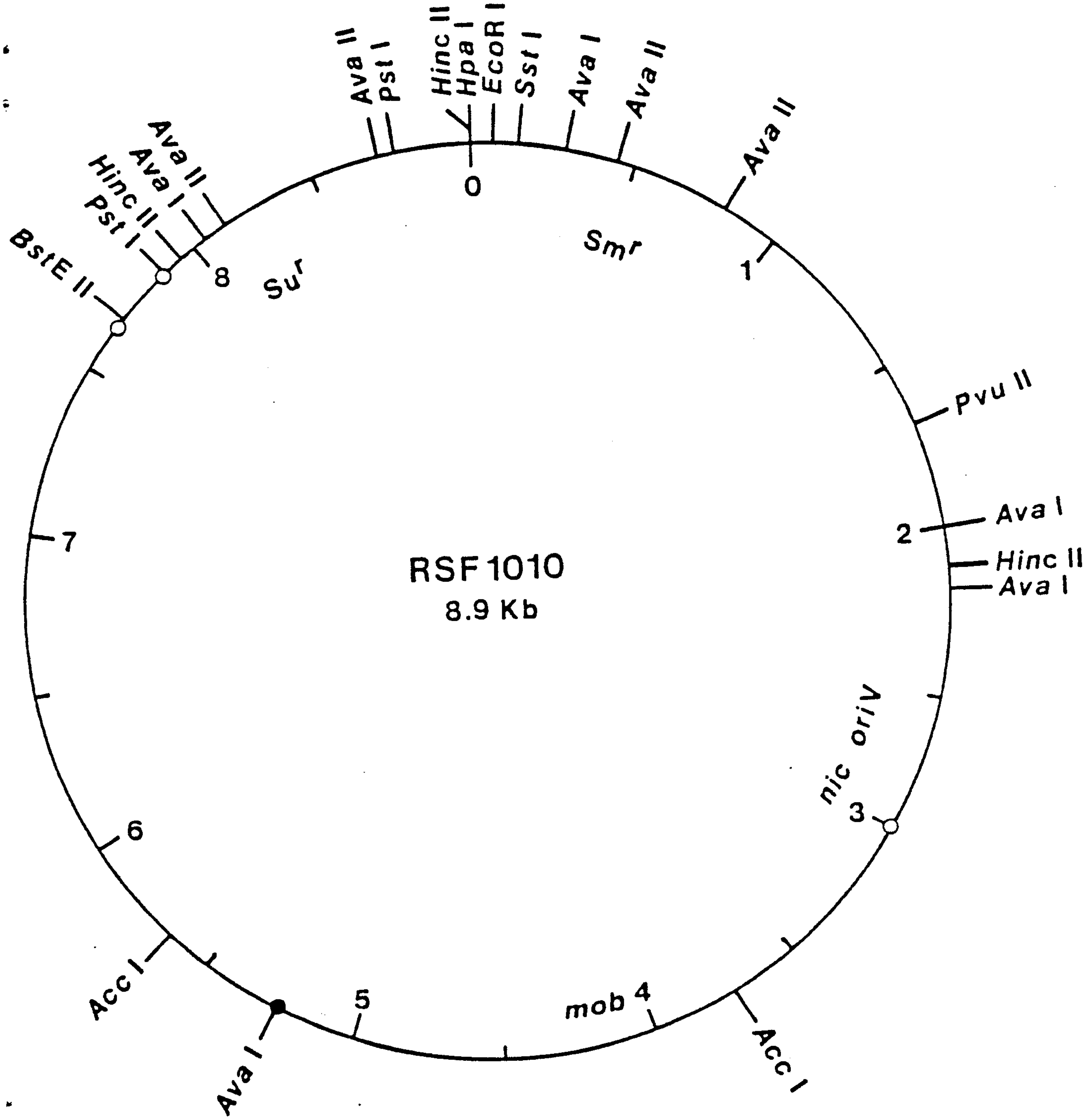


**RSF 1010:** PLASMID MIT BREITER WIRTSSPEZIFITAET
Erkennungsstellen der Endonukleasen

Figur 5

GRUPPE 1 SORBOSE OXIDASE GEN-REGION

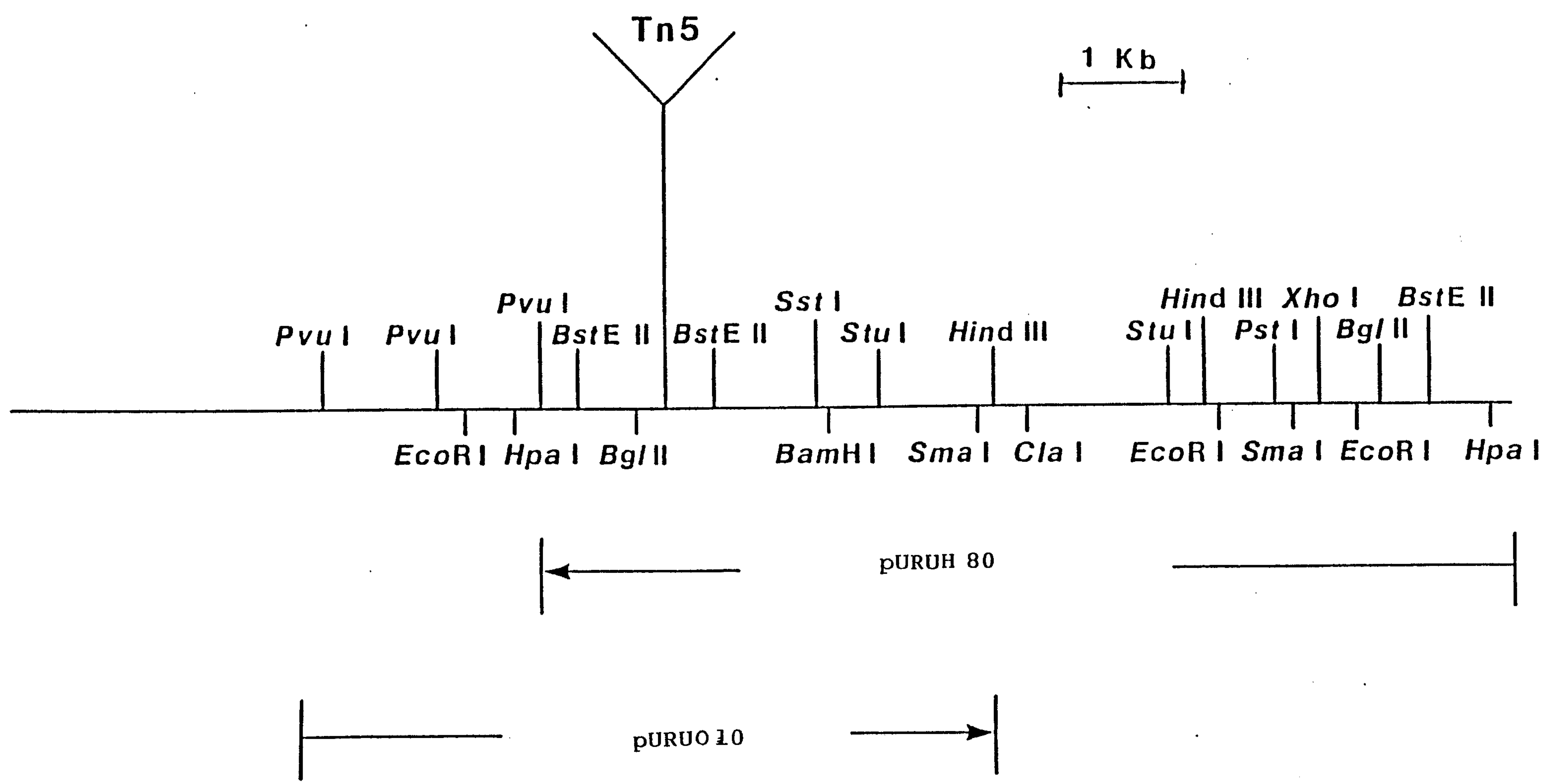

Figur 6

GRUPPE 2 SORBOSE OXIDASE
GEN-REGION